# EUROPÄISCHE PATENTANMELDUNG

(11) **EP 3 799 833 A1**
(43) Veröffentlichungstag der Anmeldung: **07.04.2021**
(21) Anmeldenummer: 20197426.8
(22) Anmeldetag: 22.09.2020
(51) Int. Cl.: A61F 2/12, A61F 2/00

(54) **HÜLLE FÜR EIN IMPLANTAT UND UMHÜLLTES IMPLANTAT FÜR WEICHGEWEBE**

(30) Priorität: 02.10.2019 DE 102019126600
(71) Anmelder: Neumann, Hans-Georg, 18146 Rostock (DE)
(72) Erfinder: Neumann, Hans-Georg, 18146 Rostock (DE)
(74) Vertreter: Prinz & Partner mbB

(57) **Zusammenfassung**

Eine Hülle (10) für ein Implantat (24) ist gebildet aus einer ersten und einer zweiten Platte (12, 14), wobei die zweite Platte (14) mittels einer Naht (18) in einem Randbereich der ersten Platte (12) an dieser befestigt ist. Die erste Platte (12) und die zweite Platte (14) bestehen aus einer zellfreien Kollagenmatrix.

Ferner wird ein umhülltes Implantat (22) für Weichgewebe, bevorzugt ein Brustimplantat, mit einer solchen Hülle (10) angegeben.

## Beschreibung

Die Erfindung betrifft eine Hülle für ein Implantat sowie ein umhülltes Implantat für Weichgewebe mit einer derartigen Hülle.

In der chirurgischen Behandlung von Erkrankungen sowie Verletzungen stehen eine Vielzahl von Implantaten zur Verfügung, die als Ersatz von krankheitsbedingt entferntem Gewebe zum Einsatz kommen können. Beispielsweise kann mittels Silikonimplantaten nach einer erfolgten Mastektomie eine Brustrekonstruktion erfolgen.

Aufgrund des Einsatzes innerhalb des menschlichen Körpers bestehen hinsichtlich der Verträglichkeit und der Langzeitstabilität der eingesetzten Implantate hohe Anforderungen.

Zugleich sollte die Benutzung der Implantate möglichst einfach und zuverlässig erfolgen können, um Operationszeiten zu minimieren und Komplikationen zu vermeiden.

Aus der WO 2014/041577 A1 ist eine schachtelartige Umhüllung für ein Brustimplantat bekannt, die in der Rekonstruktion der Brust genutzt werden kann. Zur besseren Verträglichkeit ist die Umhüllung aus einem Biomaterial gefertigt, das im Zuge des Heilungsprozesses in körpereigenes Gewebe umgewandelt wird. Die Umhüllung liegt zunächst als flaches Blatt mit mehreren Sektionen bzw. Segmenten mit spezifischer Geometrie vor. Während der Operation wird das Brustimplantat auf das noch flache Blatt gelegt, dieses anschließend zur schachtelartigen Umhüllung geformt und in dieser Form vernäht und eingesetzt. Dadurch ist jedoch der Einsatz der Umhüllung mit einem hohen Zeitaufwand während der Operation verbunden.

Die Aufgabe der Erfindung ist es, ein bioverträgliches Implantat zur Verfügung zu stellen, das einfach in der Anwendung ist.

Die Aufgabe wird erfindungsgemäß gelöst durch eine Hülle für ein Implantat, wobei die Hülle aus einer ersten und einer zweiten Platte gebildet ist. Die zweite Platte ist mittels einer Naht in einem Randbereich der ersten Platte an dieser befestigt. Die erste Platte und die zweite Platte bestehen erfindungsgemäß aus einer zellfreien Kollagenmatrix.

Die Hülle sorgt für eine ausreichende Stabilität des Implantats direkt nach dem Einsetzen. Zugleich kann das Implantat über die Hülle mit dem umgebenden Gewebe vernäht und sicher fixiert werden. Weiterhin kann die Hülle wenigstens teilweise an die Form des Implantats angepasst werden.

Zellfreie Kollagenmatrices sind nicht zytotoxisch und eignen sich daher in besonderem Maße als Material für den Einsatz als Hülle des Implantates. Des Weiteren wird die Kollagenmatrix vom Körper als eigenes Gewebe erkannt und während des natürlichen Heilungsprozesses in das umgebende Gewebe eingebaut. Somit kann die Hülle den Heilungsprozess aktiv unterstützen.

Durch die Verwendung der Kollagenmatrix sind die erste und zweite Platte und somit auch die Hülle als solche zudem flexibel und in Wasser quellbar. Die Hülle kann daher einfach verarbeitet werden und sich an die Form des zu umhüllenden Implantats anpassen. Dadurch eignet sich die Hülle in besonderem Maße dazu, für Implantate unterschiedlicher Größe zum Einsatz zu kommen.

Die Naht im Randbereich der ersten Platte stellt eine einfache Möglichkeit zum Fixieren der zweiten an der ersten Platte dar. Zudem sind eine Vielzahl von biokompatiblen Materialien, wie zum Beispiel Nahtmaterial aus azellulären dermalen oder pericardialen Matrizen, bekannt, die als Garn für die Naht genutzt werden können. Da die Hülle im Zuge des Heilungsprozesses in das umgebende Gewebe eingebaut wird, können zudem vom Körper resorbierbare Garne genutzt werden. Bevorzugt ist die Naht ebenfalls aus einer zellfreien Kollagenmatrix gebildet, wie sie auch zur Herstellung der ersten und zweiten Platte genutzt wird.

Dadurch, dass die erste und die zweite Platte nur in einem Randbereich aneinander befestigt sind, wird zwischen den Platten ein Zwischenraum geschaffen, in den das Implantat aufgenommen werden kann.

Die erste und die zweite Platte können einen runden Querschnitt aufweisen, bevorzugt einen ovalen oder kreisförmigen Querschnitt. Grundsätzlich kann die Form der Hülle an die Form des zu umhüllenden Implantats und/oder die angedachte Anwendung angepasst sein. Ein runder Querschnitt eignet sich beispielsweise in besonderem Maß für Brustimplantate.

Der Querschnitt der ersten Platte kann sich auch vom Querschnitt der zweiten Platte unterscheiden.

Die erste und die zweite Platte können einen Durchmesser im Bereich von 10 bis 20 cm aufweisen, insbesondere einen Durchmesser im Bereich von 13 bis 17 cm. Grundsätzlich sollte der Durchmesser der Hülle an die Größe des zu umhüllenden Implantats angepasst sein, um eine korrekte Positionierung des Implantats in der Hülle zu gewährleisten.

In einer Ausführungsform weist die zweite Platte eine Aussparung auf, insbesondere eine runde Aussparung, mit einem Durchmesser von 4 bis 8 cm. Die runde Aussparung ist insbesondere eine ovale oder kreisförmige Aussparung.

Die Aussparung ermöglicht es, das Implantat in die Hülle einzusetzen, auch nachdem die zweite Platte an der ersten Platte vernäht worden ist. Somit muss das Vernähen nicht während einer Operation durchgeführt werden, sodass die Operationszeit für das Einsetzen des Implantats reduziert wird. Gleichzeitig kann das passende Implantat weiterhin vor Ort während der Operation ausgewählt und vorbereitet werden, sodass eine flexible Verwendung der Hülle gewährleistet bleibt.

Dadurch, dass die erste Platte und die zweite Platte aus einer Kollagenmatrix bestehen und somit die gesamte Hülle flexibel ist, kann die Öffnung kurzzeitig so weit vergrößert werden, dass das Implantat zwischen der ersten Platte und der zweiten Platte eingesetzt werden kann. Anschließend zieht sich die Öffnung nach Einsetzen des Implantats wenigstens teilweise wieder zusammen, sodass das Implantat innerhalb der Hülle sicher fixiert bleibt.

Das Auseinanderziehen der Öffnung ist dabei ohne übermäßigen Kraftaufwand auch per Hand möglich.

In einer weiteren Ausführungsform können die erste und die zweite Platte im Randbereich einen Abschnitt aufweisen, in dem sie nicht miteinander vernäht sind. Der nahtfreie Abschnitt im Randbereich bildet somit eine Öffnung nach Art eines Sacklochs, durch die das Implantat in den Zwischenraum zwischen der ersten und der zweiten Platte eingeführt werden kann.

Die erste und die zweite Platte können eine Dicke im Bereich von 0,2 bis 2 mm aufweisen, insbesondere von 0,5 bis 1,5 mm. Eine geringere Dicke der Hülle kann deren Stabilität reduzieren, während eine höhere Dicke die Elastizität der Hülle reduzieren kann und mehr Material benötigt.

Die erste und die zweite Platte können auch unterschiedliche Dicken aufweisen. Somit ist eine Anpassung an die angedachte Position im Körper sowie an Anforderungen hinsichtlich der später zu erwartenden Belastungen, beispielsweise bei Bewegung, möglich.

Die Naht kann ein Heftstich, ein Rückstich oder ein Reihstich sein. Eine solche Naht gewährleistet eine sichere Verbindung und ist besonders einfach anzubringen. Insbesondere kann eine derartige Naht auch maschinell angebracht werden.

In einer Variante ist die Naht ein Überwendlichstich. Eine derartige Naht gewährleistet ebenfalls eine sichere Verbindung zwischen erster und zweiter Platte. Zusätzlich werden jedoch im Vergleich zum Heftstich überstehende Randbereiche der Hülle reduziert oder beseitigt, da das Garn zumindest teilweise über einen Außenrand der ersten und der zweiten Platte geführt wird. Mit anderen Worten werden die erste und die zweite Platte formschlüssiger zusammengefügt als es beispielsweise mit einem Heftstich möglich ist. Dies ermöglicht eine glattere Form des mit der Hülle versehenen Implantats und eine einfachere Anbringung der Hülle während einer Operation.

Die Kollagenmatrix ist bevorzugt aus zellfreier Spalthaut gebildet, die gute Quelleigenschaften sowie eine leichte Formbarkeit aufweist. Die zellfreie Spalthaut kann beispielsweise aus Schweinehaut gewonnen werden.

Das Implantat ist insbesondere ein Weichteilimplantat, bevorzugt ein Brustimplantat. Die erfindungsgemäße Hülle ist durch deren Flexibilität in besonderem Maße an ein Weichteilimplantat anpassbar, während zugleich eine sichere Fixierung des Weichteilimplantats über die Hülle erzielt wird.

Die Aufgabe wird des Weiteren gelöst durch ein umhülltes Implantat für Weichgewebe, bevorzugt ein umhülltes Brustimplantat, mit einer Hülle der zuvor beschriebenen Art.

Weitere Vorteile und Eigenschaften der Erfindung ergeben sich aus der nachfolgenden Beschreibung beispielhafter Ausführungsformen und den Zeichnungen, auf die Bezug genommen wird. In diesen zeigen:
- Figur 1 eine erste Ausführungsform einer erfindungsgemäßen Hülle;
- Figur 2 ein erfindungsgemäßes umhülltes Implantat mit der Hülle aus Figur 1 in einem Querschnitt;
- Figur 3 eine zweite Ausführungsform der erfindungsgemäßen Hülle; und
- Figur 4 eine dritte Ausführungsform der erfindungsgemäßen Hülle.

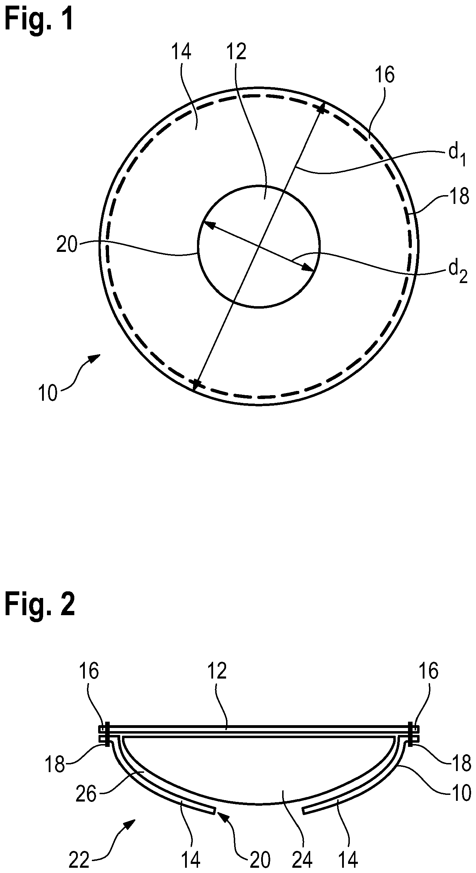

In Figur 1 ist eine erste Ausführungsform einer erfindungsgemäße Hülle 10 dargestellt, mit einer ersten Platte 12 sowie einer zweiten Platte 14.

Die erste Platte 12 und die zweite Platte 14 sind in einem Randbereich am Außenrand der ersten Platte 12 unter Bildung eines Rands 16 mittels einer Naht 18 verbunden. Mit anderen Worten ist die zweite Platte 14 an der ersten Platte 12 in Radialrichtung außen mittels der Naht 18 befestigt.

Die Naht 18 ist in der ersten Ausführungsform ein Heftstich.

Die erste Platte 12 und die zweite Platte 14 sind kreisförmig ausgebildet und weisen den gleichen Durchmesser d₁ von 15 cm auf. Grundlegend kann der Durchmesser d₁ im Bereich von 10 bis 20 cm liegen, insbesondere von 13 bis 15 cm.

Auch können sich die Durchmesser d₁ der ersten Platte 12 und der zweiten Platte 14 voneinander unterscheiden, insbesondere kann die zweite Platte 14 einen geringeren Durchmesser d₁ aufweisen als die erste Platte 12.

Die zweite Platte 14 weist eine runde Aussparung 20 auf, mit einem Durchmesser d₂ von 7 cm. Die Aussparung 20 ist in Radialrichtung innen, bevorzugt mittig, in der zweiten Platte 14 angeordnet. Der Durchmesser d₂ der Aussparung 20 kann grundlegend im Bereich von 4 bis 8 cm liegen. Insbesondere beträgt der Durchmesser d₂ der Aussparung 20 etwa 30 bis 50 % des Durchmessers d₁ der zweiten Platte 14.

In der in Figur 1 gezeigten Ausführungsform hat die runde Aussparung 20 einen kreisförmigen Querschnitt.

Sowohl die erste Platte 12 als auch die zweite Platte 14 weisen eine Dicke im Bereich von 0,2 bis 2 mm auf, insbesondere von 0,5 bis 1,5 mm.

Die erste Platte 12 und die zweite Platte 14 bestehen aus einer zellfreien Kollagenmatrix, sodass die gesamte Hülle 10 flexibel ist und in Wasser aufquellen kann.

Die Kollagenmatrix ist vorzugsweise eine zellfreie Spalthaut, besonders bevorzugt eine aus Schweinehaut gewonnene zellfreie Spalthaut.

In Figur 2 ist eine schematische Darstellung einer Ausführungsform eines erfindungsgemäßen umhüllten Implantats 22 im Querschnitt gezeigt, das ein Implantat 24 sowie die Hülle 10 aus Figur 1 umfasst. Der Querschnitt in Figur 2 verläuft entlang einer Linie, die der in Figur 1 gezeigten Linie d₁ der Hülle 10 entspricht.

Das Implantat 24 ist in der gezeigten Ausführungsform ein Silikonkissen und das umhüllte Implantat 22 ein Brustimplantat.

Aus Figur 2 wird ersichtlich, dass das Implantat 24 in einem Zwischenraum 26 zwischen der ersten Platte 12 und der zweiten Platte 14 angeordnet ist. Der Durchmesser d₂ der Aussparung 20 ist dabei so gewählt, dass das Implantat 24, sobald es in die Hülle 10 eingesetzt worden ist, nicht aus der Hülle 10 herausrutschen kann.

Der Durchmesser d₂ der Aussparung 20 der Hülle 10 kann nach dem Einsetzen des Implantats 24 dauerhaft größer sein als vor dem Einsetzen des Implantats 24. Entscheidend ist vor allem, dass das Herausrutschen des Implantats 24 aus der Hülle 10 verhindert wird.

In Figur 2 ist zu erkennen, dass der Rand 16 auch nach dem Einsetzen des Implantats 24 radial nach außen absteht. Der Rand 16 kann beispielsweise zum Fixieren des umhüllten Implantats 22 am Gewebe eines Patienten genutzt werden.

Im Folgenden wird die Funktionsweise der Hülle 10 erläutert.

Zur Herstellung des umhüllten Implantats 22, wird zunächst die Hülle 10 für einige Zeit in sterile Kochsalzlösung, Ringer- oder Ringer-Lactat-Lösung gelegt, beispielsweise für 5 bis 10 Minuten.

Da die erste Platte 12 sowie die zweite Platte 14 aus einer Kollagenmatrix bestehen, nimmt die Hülle 10 die Lösung auf und quillt daher auf. Zudem erhöht sich durch die Lösungsaufnahme die Formbarkeit der Hülle 10.

Dies ermöglicht es, die Aussparung 20, beispielweise per Hand oder mittels geeignetem OP-Besteck, so weit zu dehnen, dass das Implantat 24 durch die ausgedehnte Aussparung 20 in die Hülle 10 eingesetzt werden kann.

Anschließend zieht sich die Aussparung 20 wenigstens teilweise wieder zusammen, sodass das Implantat 24 nicht von alleine aus der Hülle 10 herausrutschen kann.

Das so erhaltene umhüllte Implantat 22 kann anschließend direkt verwendet werden, in der gezeigten Ausführungsform als Brustimplantat.

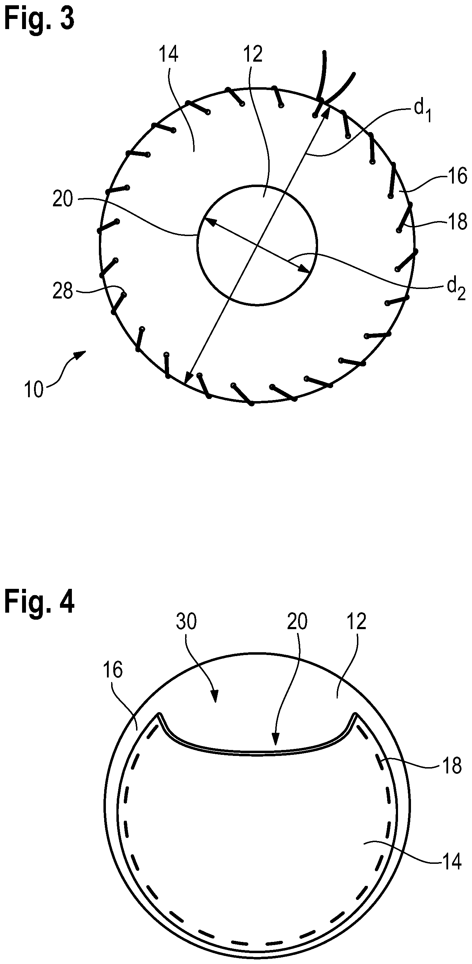

In Figur 3 ist eine zweite Ausführungsform der erfindungsgemäßen Hülle 10 gezeigt.

Die zweite Ausführungsform der Hülle 10 entspricht im Wesentlichen der ersten Ausführungsform, sodass im Folgenden lediglich auf die Unterschiede eingegangen wird. Gleiche und funktionsgleiche Teile werden mit denselben Bezugszeichen versehen, sodass auf die obigen Erläuterungen verwiesen wird.

In der zweiten Ausführungsform ist die Naht 18 ein Überwendlichstich. Entsprechend verläuft die Naht 18, wie in Figur 3 gezeigt, teilweise entlang der Außenkante der ersten Platte 12 und der zweiten Platte 14.

Zudem weisen die erste Platte 12 und die zweite Platte 14 Löcher 28 auf, die kreisförmig angeordnet sind und durch die das Garn der Naht 18 geführt ist.

Die Löcher 28 sind in der gezeigten Ausführungsform in radialer Richtung 1 cm entfernt von der Außenkante der ersten Platte 12 beziehungsweise der zweiten Platte 14 angeordnet, sodass die Löcher 28 ebenfalls kreisförmig angeordnet sind.

Durch die Verwendung eines Überwendlichstichs als Naht 18 kann die Größe des radial nach außen abstehenden Rands 16 im Vergleich zur in Figur 2 gezeigten Ausführungsform des umhüllten Implantats 22 deutlich reduziert werden. Dies kann eine günstigere Passform für den Einsatz in einem Patienten ergeben und somit den Aufwand beim Einsetzen des umhüllten Implantats 22 weiter reduzieren.

In Figur 4 ist eine dritte Ausführungsform der erfindungsgemäßen Hülle 10 gezeigt.

Die dritte Ausführungsform der Hülle 10 entspricht im Wesentlichen den vorherigen Ausführungsformen, sodass im Folgenden lediglich auf die Unterschiede eingegangen wird. Gleiche und funktionsgleiche Teile werden mit denselben Bezugszeichen versehen, sodass auf die obigen Erläuterungen verwiesen wird.

In der dritten Ausführungsform der Hülle 10 weisen die erste und die zweite Platte 12, 14 einen Abschnitt 30 auf, in dem sie nicht miteinander vernäht sind.

Dieser nahtfreie Abschnitt 30 ist im Randbereich der ersten und zweiten Platte 12, 14 angeordnet. Somit wird die Aussparung 20 radial außen liegend, im Bereich des Randes 16 der ersten und zweiten Platte 12, 14 gebildet.

Das Implantat 24 kann über diese Aussparung 20 in den Zwischenraum 26 zwischen der ersten und der zweiten Platte 12, 14 eingeführt werden.

Durch die Verwendung einer vorgefertigten Hülle 10 aus zellfreier Kollagenmatrix, die aus in einem Randbereich miteinander vernähten ersten und zweiten Platten 12, 14 gebildet ist, können die im Stand der Technik bekannten vorteilhaften Eigenschaften einer Kollagenhülle beibehalten und die Operationszeiten deutlich verkürzt werden.

## Patentansprüche

1. Hülle für ein Implantat (24), wobei die Hülle (10) aus einer ersten und einer zweiten Platte (12, 14) gebildet ist, wobei die zweite Platte (14) mittels einer Naht (18) in einem Randbereich der ersten Platte (12) an dieser befestigt ist und wobei die erste Platte (12) und die zweite Platte (14) aus einer zellfreien Kollagenmatrix bestehen.

2. Hülle nach Anspruch 1, **dadurch gekennzeichnet, dass** die erste und die zweite Platte (12, 14) einen runden Querschnitt aufweisen, bevorzugt einen ovalen oder kreisförmigen Querschnitt.

3. Hülle nach Anspruch 1 oder 2, **dadurch gekennzeichnet, dass** die erste und die zweite Platte (12, 14) einen Durchmesser d₁ im Bereich von 10 bis 20 cm aufweisen, insbesondere von 13 bis 17 cm.

4. Hülle nach einem der vorhergehenden Ansprüche, **dadurch gekennzeichnet, dass** die zweite Platte (14) eine Aussparung (20) aufweist, insbesondere eine runde Aussparung (20) mit einem Durchmesser d₂ von 4 bis 8 cm.

5. Hülle nach einem der Ansprüche 1 bis 3, **dadurch gekennzeichnet, dass** eine Aussparung (20) vorgesehen ist, die durch einen nahtfreien Abschnitt (30) im Randbereich der ersten und zweiten Platte (12, 14) gebildet ist.

6. Hülle nach einem der vorhergehenden Ansprüche, **dadurch gekennzeichnet, dass** die erste und die zweite Platte (12, 14) eine Dicke im Bereich von 0,2 bis 2 mm aufweisen, insbesondere von 0,5 bis 1,5 mm.

7. Hülle nach einem der vorhergehenden Ansprüche, **dadurch gekennzeichnet, dass** die Naht (18) ein Heftstich, ein Rückstich oder ein Reihstich ist.

8. Hülle nach einem der vorhergehenden Ansprüche, **dadurch gekennzeichnet, dass** die Naht (18) ein Überwendlichstich ist.

9. Hülle nach einem der vorhergehenden Ansprüche, **dadurch gekennzeichnet, dass** die Kollagenmatrix zellfreie Spalthaut ist.

10. Hülle nach einem der vorhergehenden Ansprüche, **dadurch gekennzeichnet, dass** das Implantat (24) ein Weichteilimplantat ist, bevorzugt ein Brustimplantat.

11. Umhülltes Implantat (22) für Weichgewebe, bevorzugt ein Brustimplantat, mit einer Hülle (10) gemäß einem der vorhergehenden Ansprüche
